# EUROPEAN PATENT APPLICATION

(11) **EP 4 714 383 A1**
(43) Date of publication of application: **25.03.2026**
(21) Application number: 24201013.0
(22) Date of filing: 18.09.2024
(51) Int. Cl.: A61B 18/20, H05B 41/30

(54) **A DEVICE FOR HAIR REMOVAL OR TREATMENT OF SKIN**

(71) Applicant: Koninklijke Philips N.V., 5656 AG Eindhoven (NL)
(72) Inventor: VAN GOMPEL, Antonius Petrus Andreas Maria, 5656 AG Eindhoven (NL)
(74) Representative: Philips Intellectual Property & Standards

(57) **Abstract**

A device for hair removal or treatment of skin comprises a light source, a capacitor for storing charge for delivery to the light source for generating a flash of treatment light and a power source for charging the capacitor. A switching circuit controls the charging of the capacitor from the power source. It has a low side switch and a first unidirectional current flow component in series for carrying a charging current, and a second unidirectional current flow component second of opposite polarity for carrying a discharging current. In this way, the discharging current does not pass through the switch despite having a low side topology.

## Description

### FIELD OF THE INVENTION

This invention relates to a device for hair removal or treatment of skin, in particular by applying treatment light to the skin.

### BACKGROUND OF THE INVENTION

For hair removal, photo-epilation devices are well-known. People use hair epilators or depilators to remove unwanted hair. Typical target areas for women are the face, armpit, arm, leg, bikini line, and body. Men use light-based epilators also on the chest and back.

Photo-epilation devices are based on the filtered output of a flashlamp, known as Intense Pulsed Light, IPL.

The energy required for generating the flash of the flashlamp (i.e., an optical pulse) in IPL products is stored in a capacitor, known as a flash capacitor. Three states can be identified related to the flash capacitor; a charging state, a discharging state, and a charged state.

In the short duration discharging state, which for example lasts tens of ms, the energy required for the optical pulse is transferred from the flash capacitor to the lamp for the duration of the optical pulse. High voltages (several hundred Volts) are required to generate this optical pulse and a large current (approximately 100 to 200 Amps) flows during the optical pulse.

Before the discharge can take place, the flash capacitor is charged during the charging state. Depending on the operating principles (such as pulse cutting or free decay) the flash capacitor is charged to either a fixed voltage level or to a variable voltage level. A high voltage source is used to generate the energy and charge the flash capacitor. Depending on the high voltage source and other design choices, the charging of the flash capacitor can be done by enabling/disabling the high voltage source, or by having a dedicated switch that controls the charging of the flash capacitor.

In the charged state, the capacitor is charged to a certain voltage but is not being actively charged or discharged and is maintaining its voltage.

This disclosure relates to designs in which a dedicated switch is used to charge the flash capacitor. In theory, this switch can be placed along the positive line of the flash capacitor, the so-called high side, or it can be placed along the negative line of the flash capacitor, the so-called low side. Both high side and low side switching are well known.

Typically, a high side switch is used. The advantage of high side charging is that only the relatively low current needed for charging the flash capacitor needs to be switched. The disadvantage is that the control signal from a microcontroller/processor needs to be translated from a low voltage to a high voltage for controlling the switch.

The use of a low side switch also has some disadvantages. For one, the switch needs to be able to handle both the charging and discharging current. Another disadvantage is the fact that the top side of the switch will be at a negative potential during the discharge (as a result of a voltage drop across the switch from one terminal which is grounded to the other terminal). The major advantage is that the translation to a high voltage, in order to control the switch, is not required and thus a cost saving can be obtained.

There remains a need to enable the cost benefits of low side switching but without the associated disadvantages explained above.

### SUMMARY OF THE INVENTION

The invention is defined by the claims.

According to examples in accordance with an aspect of the invention, there is provided a device for hair removal or treatment of skin, comprising:
a light source for generating a flash of treatment light, the light source having a high side connection and a low side connection;
a capacitor for storing charge for energy delivery to the light source for generating the flash of treatment light, the capacitor having a high side terminal and a low side terminal;
a power source for charging the capacitor, the power source having an output connected to the high side terminal, wherein the high side terminal is connected to the high side connection; and
a switching circuit for controlling the charging of the capacitor from the power source,
wherein the switching circuit comprises:
   a first unidirectional current flow component and a first switch in series between the low side terminal and a ground terminal, for allowing current to flow from the low side terminal to the ground terminal during charging of the capacitor;
   a second unidirectional current flow component in parallel with the first unidirectional current flow component and the first switch, with opposite flow direction to the first unidirectional current flow component.

This charging circuit uses a low side switch. The switching circuit addresses the disadvantages that are implicitly obtained by using a low side switch topology, namely (i) the high current capability requirement due to the large capacitor discharge current and (ii) the negative potential on the node connecting the low side switch to the flash capacitor. This negative potential results from voltage drops across the low side switch. The unidirectional current flow components address these issues.

The switching circuit may also be used for controlling the discharging of the capacitor to the light source, and in that case comprises a second switch between the low side connection and the ground terminal. The first switch provides a capacitor charging current path, and the optional second switch provides a capacitor discharging current path that bypasses the switch.

The first and second unidirectional current flow components for example each comprise a diode. Such diodes are already used in existing IPL products, so they may be re-arranged without resulting in a cost increase. For example, diodes are used to charge the positive side of the lamp to the operating voltage (e.g. 400V), independent of the voltage of the flash capacitor, in a high side circuit. This 400V is used during the ignition of the lamp to create 800V across the lamp during the so-called boost stage.

If the circuit topology is changed from high side to low side switching, these diodes are rendered obsolete so that they may be used as the unidirectional current flow components outlined above.

The arrangement provides a solution which is particularly applicable to IPL devices because the IPL circuit requires a low current to be switched on and off in one direction (the charging direction) and requires a high current to flow in the other direction (the discharging direction).

The power source is for example a constant power source. It may however be a voltage source or a current source.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an IPL device;
Fig. 2 shows a first known IPL circuit with a high side switch;
Fig. 3 shows an IPL circuit of this disclosure;
Fig. 4 shows timing diagrams for charging; and
Fig. 5 shows timing diagrams for discharging.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device for hair removal or treatment of skin comprising a light source, a capacitor for storing charge for delivery to the light source for generating a flash of treatment light and a power source for charging the capacitor. A switching circuit controls the charging of the capacitor from the power source and discharging of the capacitor to the light source. Different switches of the switching circuit perform the charging and discharging functions. The switching circuit has a low side switch and a first unidirectional current flow component in series for carrying a charging current, and a second unidirectional current flow component second of opposite polarity for carrying a discharging current. In this way, the discharging current does not pass through the switch despite having a low side topology.

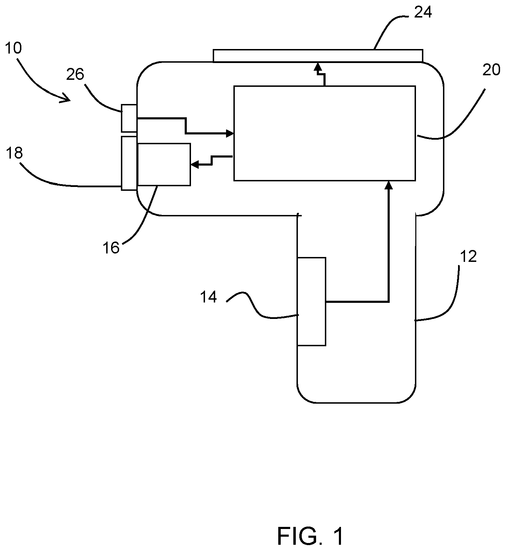

Fig. 1 shows an IPL device 10, in the form of a hand-held device having a handle 12 with a trigger 14, an IPL light source 16 providing light through a light delivery window 18. A controller 20 controls the light source 16 to deliver light flashes to the light delivery window 18.

The device of Fig. 1 also has an output interface 24, such as a screen and/or loudspeaker. The output interface to the user may instead or additionally be provided by a remote device with which the hand-held device communicates.

Optionally, a skin sensor 26 is used to detect skin contact. It may comprise a single sensor, or two or more sensors to detect that the light output window is flat against the skin.

In use of the device, a user applies the light output window to the skin and presses the trigger 14 to deliver a pulse of light. The user then slides the device over the skin to the next treatment location and applies the trigger to deliver the next flash of light. It is also possible in some devices to keep the trigger depressed during the line treatment. It will then automatically flash every time a capacitor in the device is charged enough, which is typically 1-2 seconds depending on the setting. The user may then just move the device to the next spot, wait for the next flash, and move on again.

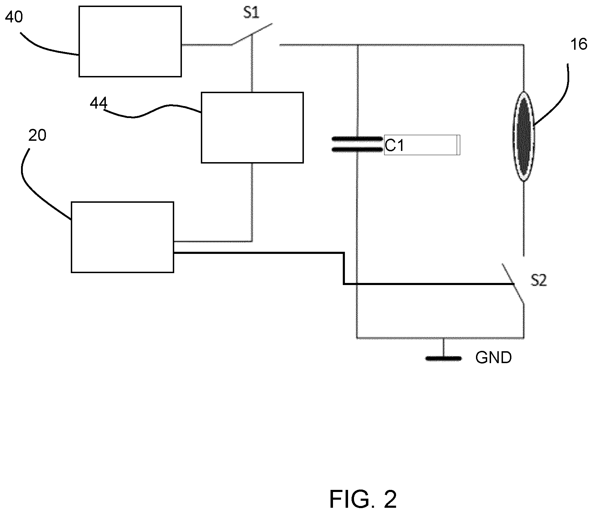

Fig. 2 shows a first known IPL circuit with a high side switch. The circuit comprises a power source 40 and a first, high side, switch S1 for delivery of energy (as current flow) from the power source 40 to the capacitor C1 during capacitor charging. The capacitor C1 is connected between an output side of the switch S1 and a ground terminal GND.

The controller 20 controls the switch S1 to close during charging and open during discharging, i.e., flash delivery.

In the particular implementation shown, a second switch S2 is in series with the light source 16 and is also controlled by the controller 20. The controller 20 controls the switch S2 to open during capacitor charging and close during discharging, i.e., flash delivery.

When not ignited the lamp has a very high impedance and no current can flow. The ignition is started by a very high voltage (for example of the order of 10kV) close to the lamp gas (for example using an electrode in the reflector) as well as a high voltage for example around 800V) on the lamp electrodes. The switch S2 applies the high voltage to the lamp electrodes. It allows the lamp current to be stopped and also enables pulse shaping. However, other switch configurations may be used to provide lamp ignition. The lamp cathode may then be grounded, either directly or via a diode.

Because a high side switch S1 is used, a level shifting circuit 44 is provided between the controller and the first switch S1.

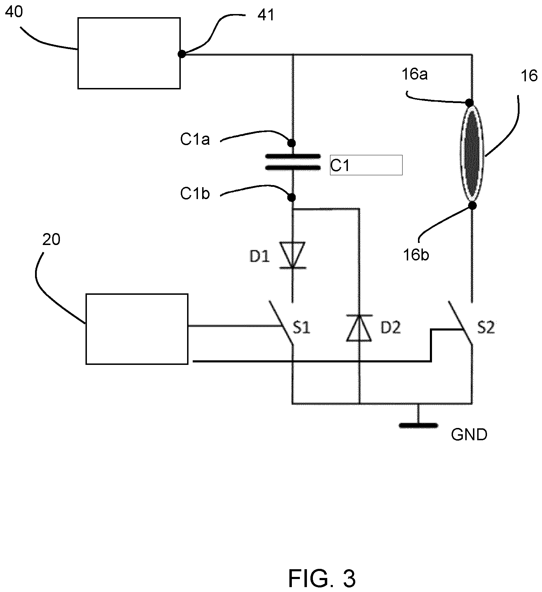

Fig. 3 shows an IPL circuit of this disclosure.

The circuit again comprises the power source 40, but a first, low side, switch S1 is used for delivery of energy from the power source 40 to the capacitor C1 during capacitor charging. The first switch S1 is now between the capacitor C1 and the ground terminal GND.

The controller 20 again controls the switch S1 to close during charging and to open when charging is complete. However, no level shifting circuit from the low voltage to the voltage level of capacitor terminal C1a is now needed.

Level shifting from the voltage of the controlling circuit (typically a microcontroller working at 1.8 to 5V) to the correct level for a transistor (MOSFET or IGBT etc., of (around 10 to 20V) might be needed. This is however a simple level conversion, referenced against ground, which is simple and cost effective to implement.

The level shifting 44 of Fig. 2 to the positive side of capacitor C1 (node C1a) is not needed in the circuit of Fig. 3, which is much more complex as it is floating between 0V and the maximum C1 voltage (300 to 400V) and therefore also more expensive.

The example shown also has the second switch S2 which is again in series with the light source 16 and is also controlled by the controller 20 to open during capacitor charging and close during discharging, i.e., flash delivery.

The light source has a high side connection 16a and a low side connection 16b.

The capacitor C1 is for storing charge for energy delivery to the light source for generating the flash of treatment light. The capacitor has a high side terminal C1a and a low side terminal C1b.

The power source 40 has an output 41 connected to the high side terminal C1a, and the high side terminal C1a is connected to the high side connection 16a, so that the charged voltage on the capacitor can be delivered to the light source.

Instead of simply using a low side switch, a switching circuit is used comprising components D1, D2 and S1.

A first diode D1 and the first switch S1 are in series between the low side terminal C1b and the ground terminal GND, for allowing current to flow from the low side terminal C1b to the ground terminal during charging of the capacitor 16.

A second diode D2 is in parallel with the first diode D1 and the first switch S1, with opposite polarity to the first diode D1. The second diode provides a bypass path around the first switch and the first diode. When the discharge current flows (through the second diode D2), a large negative voltage on the non-grounded switch terminal is avoided by the diodes D1 and D2.

The switching circuit optionally includes the second switch S2, between the low side connection 16b and the ground terminal GND.

In this circuit, the switch S1 is used for charging, and the charging current flows through the switch S1 and the first diode D1. However, the capacitor discharge current does not flow through the switch S1 because it is blocked by diode D1, and instead flows through diode D2 which bypasses the switch S1.

This relaxes the current carrying requirements of the switch S 1. In addition, during discharging, the second diode D2 prevents the low side terminal C1b of the capacitor dropping below the ground voltage by more than the diode voltage drop. The non-grounded terminal of switch S1 is also isolated by diode D1.

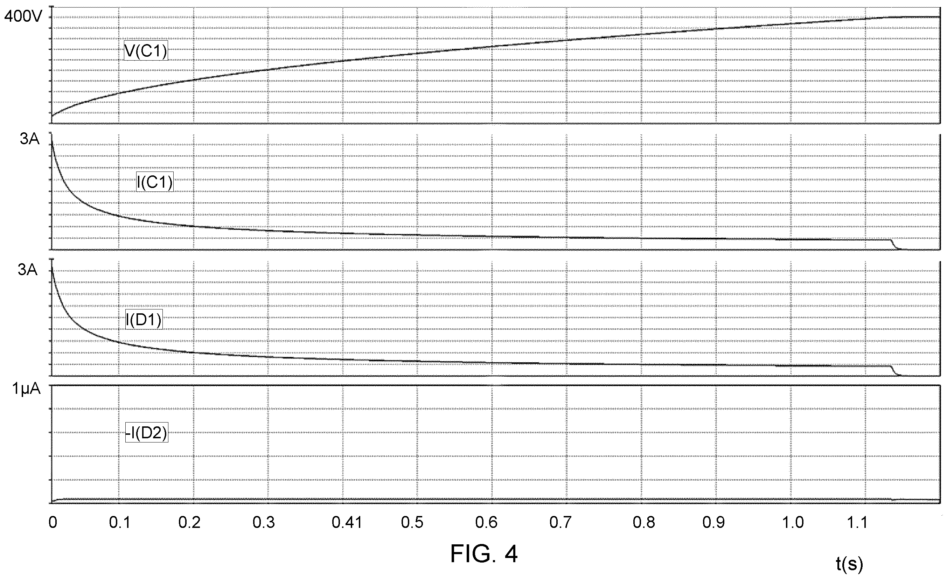

Fig. 4 shows timing diagrams for charging based on a circuit simulation.

It shows the capacitor voltage V(C1) charging to around 400 V, and the corresponding exponential decaying charging current I(C1) with an initial peak of around 3A. The charging cycle lasts around 1 second. These are just examples of possible voltage, current and timing values for the purposes of a general explanation of the circuit operation.

The current I(D1) through the first diode D1 corresponds to the capacitor charging current. There is a minimal leakage current I(D2) through the second diode.

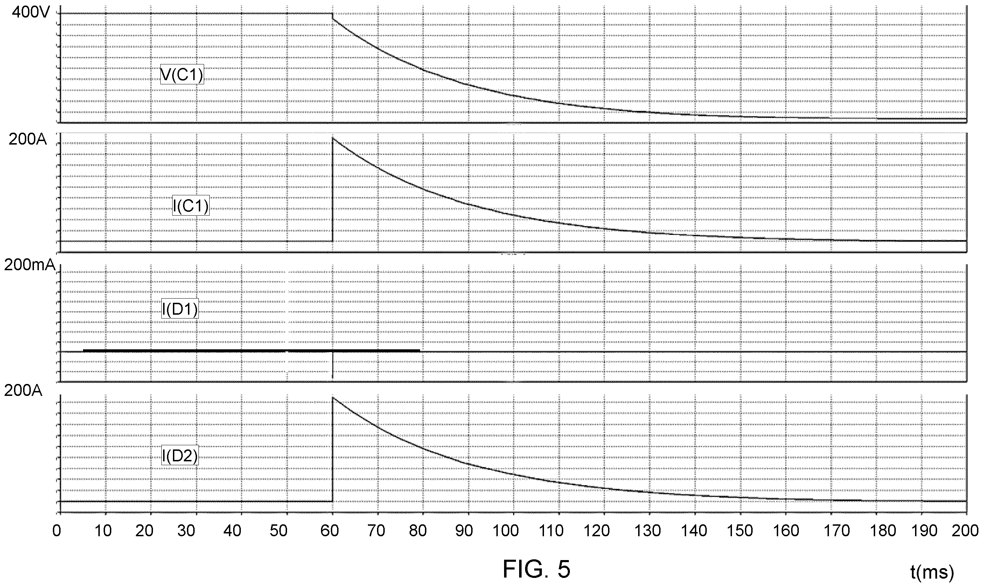

Fig. 5 shows timing diagrams for discharging, again based on a circuit simulation.

It shows the capacitor voltage V(C1) discharging from the charged level of 400V in around 150ms in this example. The corresponding exponential decaying discharging current I(C1) has an initial peak of around 200A. Again, these are just examples of possible voltage, current and timing values. For example, the discharge may in practice be only several milliseconds. In the simulation, the lamp is simulated by a resistor. The long tail is for example cut short in practice by opening switch S2.

The current I(D2) through the second diode D2 corresponds to the capacitor discharging current. There is a minimal leakage current I(D1) through the first diode.

The diodes may be replaced by any suitable unidirectional current flow component, such as diode-connected transistors.

The power source may be a constant power source, or a constant voltage source or a current source.

The switches used in the circuit preferably comprise transistor switches, such as MOSFET, BJT, or IGBT switches.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A device for hair removal or treatment of skin, comprising:
a light source (16) for generating a flash of treatment light, the light source having a high side connection (16a) and a low side connection (16b);
a capacitor (C1) for storing charge for energy delivery to the light source for generating the flash of treatment light, the capacitor having a high side terminal (C1a) and a low side terminal (C1b);
a power source (40) for charging the capacitor, the power source having an output (41) connected to the high side terminal (C1a), wherein the high side terminal (C1a) is connected to the high side connection (16a); and
a switching circuit (D1, D2, S1) for controlling the charging of the capacitor (C1) from the power source (40) and the discharging of the capacitor to the light source (16),
wherein the switching circuit comprises:
a first unidirectional current flow component (D1) and a first switch (S1) in series between the low side terminal (C1b) and a ground terminal (GND), for allowing current to flow from the low side terminal (C1b) to the ground terminal during charging of the capacitor (16); and
a second unidirectional current flow component (D2) in parallel with the first unidirectional current flow component (D1) and the first switch (S1), with opposite flow direction to the first unidirectional current flow component (D1).

2. The device of claim 1, wherein the switching circuit is also for controlling the discharging of the capacitor to the light source, and comprises a second switch (S2) between the low side connection (16b) and the ground terminal (GND).

3. The device of claim 1 or 2, wherein the first and second unidirectional current flow components each comprise a diode.

4. The device of any one of claims 1 to 3, wherein the power source is a constant power source.
